(19)

**Europäisches Patentamt**

**European Patent Office**

**Office européen des brevets**

(11) **EP 2 455 082 A1**

(12) **EUROPEAN PATENT APPLICATION**
published in accordance with Art. 153(4) EPC

(43) Date of publication:
**23.05.2012 Bulletin 2012/21**

(21) Application number: **10799940.1**

(22) Date of filing: **16.07.2010**

(51) Int Cl.:
*A61K 31/565* (2006.01)   *A61P 3/10* (2006.01)
*A61P 13/12* (2006.01)   *A61P 43/00* (2006.01)

(86) International application number:
**PCT/JP2010/062121**

(87) International publication number:
**WO 2011/007882 (20.01.2011 Gazette 2011/03)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **16.07.2009 JP 2009167695**

(71) Applicant: **Kyushu University, National University Corporation Fukuoka-shi Fukuoka 812-8581 (JP)**

(72) Inventor: **INADA, Akari Fukuoka-shi Fukuoka 812-8581 (JP)**

(74) Representative: **Vossius & Partner Siebertstrasse 4 81675 München (DE)**

(54) **INSULIN-PRODUCING CELL INDUCER, GLUCOSE INTAKE ENHANCER, AND THERAPEUTIC AGENT FOR DIABETES OR DIABETES COMPLICATIONS**

(57)   Provided is an insulin-producing cell inducer, an enhancer for glucose intake of muscles, and a therapeutic agent for diabetes or diabetes complications, wherein an active ingredient of those provided is an estrogenic compound such as estrogen and estradiol. With the present invention, functions of insulin-producing cells can be recovered since the insulin-producing cells are induced in pancreatic ducts, and blood glucose level can be immediately lowered since glucose in the blood can be effectively taken up by muscles; therefore, diabetes and diabetes complications can be prevented or treated.

EP 2 455 082 A1

**Description**

Technical Field

The present invention relates to an insulin-producing cell inducer, a glucose intake enhancer, and a therapeutic agent for diabetes or diabetes complications.

Background Art

In order to solve relative and absolute insulin insufficiency, which is a major cause of diabetes, the present inventor has focused on pancreatic insulin-producing cells, and conducted research on existing mechanisms for β-Cell insulin production (transcriptional control mechanisms for the insulin gene) .

In previous research, the present inventor has shown that a transcriptional activator and a transcriptional repressor exist in β-cells, and compete each other for insulin synthesis (transcription) (Non-Patent Literature 1). In addition, the present inventor has shown *in vitro* and *in vivo* (mouse) that, although no problems arise when the transcriptional activator and the transcriptional repressor are functioning normally, if expression of the transcriptional repressor ICER (Inducible cAMP Early Repressor) is upregulated through some effect, the function of the activator is inhibited, and insulin synthesis and growth of β-cells are suppressed, resulting in the development of diabetes symptoms due to a lack of insulin (Non-Patent Literature 2). Furthermore, the present inventor has also shown that the expression of the transcriptional repressor ICER is upregulated in a diabetic condition, and, as a result, insulin production is further reduced; and thereby the diabetes deteriorates (Non-Patent Literature 3) .

Based on these findings, a diabetes model mouse in which sufficient effects can be observed in a single line has been established (Patent Literature 1). In this model mouse, by overexpressing the transcription factor ICER in β-cells, both insulin synthesis and growth of the β-cells are suppressed, and hyperglycemia is caused in the second week after birth; and severe diabetes symptoms develop due to failure in insulin secretion. Since the hyperglycemic condition stably continues until death, a complication (diabetic nephropathy) occurs.

Citation List

Patent Literature

PTL 1: WO2004/072282

Non-patent Literature

NPL 1: The Journal of Biological Chemistry 1999, 274 (30): 21095-21103
NPL 2: Molecular and Cellular Biology 2004, 24 (7): 2831-2841
NPL 3: Biochemical and Biophysical Research Communication 1998, 253(3): 712-718

Summary of Invention

Technical Problem

An objective of the present invention is to provide a new therapeutic agent or therapeutic method for diabetes.
In addition, an objective of the present invention is to provide an effective therapeutic agent or therapeutic method for repairing sclerosed tissues of diabetic nephropathy caused by progression and aggravation of diabetes, and for regenerating healthy kidney tissues.
Furthermore, an objective of the present invention is to provide an effective therapeutic agent or therapeutic method for immediately lowering blood glucose levels.

Solution to Problem

In the model mouse in Patent Literature 1, there is a clear diabetes onset mechanism in which, by overexpressing ICER in β-cells, insulin synthesis and growth of the β-cells are suppressed; and severe diabetes symptoms develop due to failure in insulin secretion.

Surprisingly, the present inventor has found that, when estrogen is administered to this diabetes model mouse, insulin-producing cells are induced in pancreatic ducts. In addition, the present inventor found that, when estrogen is administered after the development of diabetic nephropathy symptoms, sclerosed kidney (glomeruli) tissues regenerate to healthy glomeruli. Furthermore, the present inventor has found that, by administering estrogen, expressions of molecules (AKT, Glut4, and the like) involved in glucose intake are upregulated, and glucose intake of muscle cells is enhanced.

The present invention has been accomplished based on the above-described knowledge, and includes the following embodiments.

(I) Insulin-producing cell inducer and insulin-producing cell induction method

(I-1) An insulin-producing cell inducer comprising an estrogenic compound as an active ingredient thereof.

(I-2) The insulin-producing cell inducer according to (I-1), wherein the estrogenic compound is at least one member selected from the group consisting of estradiol or esters thereof, estrone, estriol or esters thereof, equilin, ethinylestradiol, conjugated estrogens, stilbestrol, and hexestrol.

(1-3) The insulin-producing cell inducer according to (I-1), wherein the estrogenic compound is estradiol or an ester thereof.

(I-4) An insulin-producing cell induction method comprising a step of administering, to a patient with diabetes or a diabetes complication, an estrogenic compound in an effective dose to induce insulin-producing cells.

(I-5) The insulin-producing cell induction method according to (I-4), wherein the estrogenic compound is at least one member selected from the group consisting of estradiol or esters thereof, estrone, estriol or esters thereof, equilin, ethinylestradiol, conjugated estrogens, stilbestrol, and hexestrol.

(I-6) The insulin-producing cell induction method according to (I-4), wherein the estrogenic compound is estradiol or an ester thereof.

(I-7) The insulin-producing cell induction method according to any one of (1-4) to (I-6), wherein the diabetes complication is diabetic nephropathy.

(I-8) An estrogenic compound for use as an insulin-producing cell inducer in a method for preventing or treating diabetes or a diabetes complication.

(I-9) The estrogenic compound according to (I-8), wherein the estrogenic compound is at least one member selected from the group consisting of estradiol or esters thereof, estrone, estriol or esters thereof, equilin, ethinylestradiol, conjugated estrogens, stilbestrol, and hexestrol.

(I-10) The estrogenic compound according to (I-8), wherein the estrogenic compound is estradiol or an ester thereof.

(II) Glucose intake enhancer and method for enhancing glucose intake of muscles

(II-1) A glucose intake enhancer for enhancing glucose intake of muscles, the enhancer comprising an estrogenic compound as an active ingredient thereof.

(II-2) The glucose intake enhancer according to (II-1), wherein the estrogenic compound is at least one member selected from the group consisting of estradiol or esters thereof, estrone, estriol or esters thereof, equilin, ethinylestradiol, conjugated estrogens, stilbestrol, and hexestrol.

(II-3) The glucose intake enhancer according to (II-1), wherein the estrogenic compound is estradiol or an ester thereof.

(II-4) A glucose intake enhancement method for enhancing glucose intake of muscles, the method comprising a step of administering, to a patient with diabetes or a diabetes complication, an estrogenic compound in an effective dose to enhance glucose intake of muscles.

(II-5) The glucose intake enhancement method according to (II-4), wherein the estrogenic compound is at least one member selected from the group consisting of estradiol or esters thereof, estrone, estriol or esters thereof, equilin, ethinylestradiol, conjugated estrogens, stilbestrol, and hexestrol.

(II-6) The glucose intake enhancement method according to (II-4), wherein the estrogenic compound is estradiol or an ester thereof.

(II-7) The glucose intake enhancement method according to any one of (II-4) to (II-6), wherein the diabetes complication is diabetic nephropathy.

(II-8) An estrogenic compound for use as an enhancer of glucose intake of muscles in a method for preventing or treating diabetes or a diabetes complication.

(II-9) The estrogenic compound according to (II-8), wherein the estrogenic compound is at least one member selected from the group consisting of estradiol or esters thereof, estrone, estriol or esters thereof, equilin, ethinylestradiol, conjugated estrogens, stilbestrol, and hexestrol.

(II-10) The estrogenic compound according to (II-8), wherein the estrogenic compound is estradiol or an ester thereof.

(III) Therapeutic agent and therapeutic method for diabetes and a diabetes complication

(III-1) A therapeutic agent for diabetes or a diabetes complication comprising an estrogenic compound as an active ingredient thereof.

Said invention can be rephrased as: "A therapeutic agent for diabetes or a diabetes complication, containing: the insulin-producing cell inducer according to any one of (I-1) to (1-3), or the glucose intake enhancer according to any one of (II-1) to (II-3); with or without a pharmaceutically acceptable carrier or additive."

(III-2) The therapeutic agent according to (III-1), wherein the estrogenic compound is at least one member selected from the group consisting of estradiol or esters thereof, estrone, estriol or esters thereof, equilin, ethinylestradiol, conjugated estrogens, stilbestrol, and hexestrol.

(III-3) The therapeutic agent according to (III-1), wherein the estrogenic compound is estradiol or an ester thereof.

(III-4) The therapeutic agent according to any one of (III-1) to (III-3), wherein the diabetes complication is diabetic nephropathy.

(III-5) A therapeutic method for diabetes or a diabetes complication, the method comprising a step of administering, to a patient with diabetes or the diabetes complication, an estrogenic compound in a therapeutic effective dose for diabetes or the diabetes complication.

(III-6) The therapeutic method according to (III-5), wherein the estrogenic compound is at least one member selected from the group consisting of estradiol or esters thereof, estrone, estriol or esters thereof, equilin, ethinylestradiol, conjugated estrogens, stilbestrol, and hexestrol.

(III-7) The therapeutic method according to (III-5), wherein the estrogenic compound is estradiol or an ester thereof.

(III-8) The therapeutic method according to any one of (TIT-5) to (III-7), wherein the diabetes complication is diabetic nephropathy.

(III-9) An estrogenic compound for use in a method for treating diabetes or a diabetes complication.

(III-10) The estrogenic compound according to (III-9), wherein the diabetes complication is diabetic nephropathy.

Advantageous Effects of Invention

When diabetes symptoms develop, blood glucose level is controlled with exercise regimens, dietary therapies, and medication therapies. However, when a hyperglycemic condition is prolonged, β-cells are constantly pressured to produce insulin, and thereby the function and the number of the β-ceils are reduced, resulting in worsening of the diabetes.

With the present invention, the functions of insulin-producing cells can be recovered, since the insulin-producing cells are induced in pancreatic ducts.

In addition, with the present invention, blood glucose levels can be immediately lowered, since glucose in the blood can be effectively taken up by muscles.

Furthermore, with the present invention, since glomeruli that have been sclerosed due to diabetic nephropathy are regenerated back to tissues having the original elasticity, diabetic nephropathy, which has been hitherto known as difficult to treat, can be fundamentally treated.

Brief Description of Drawings

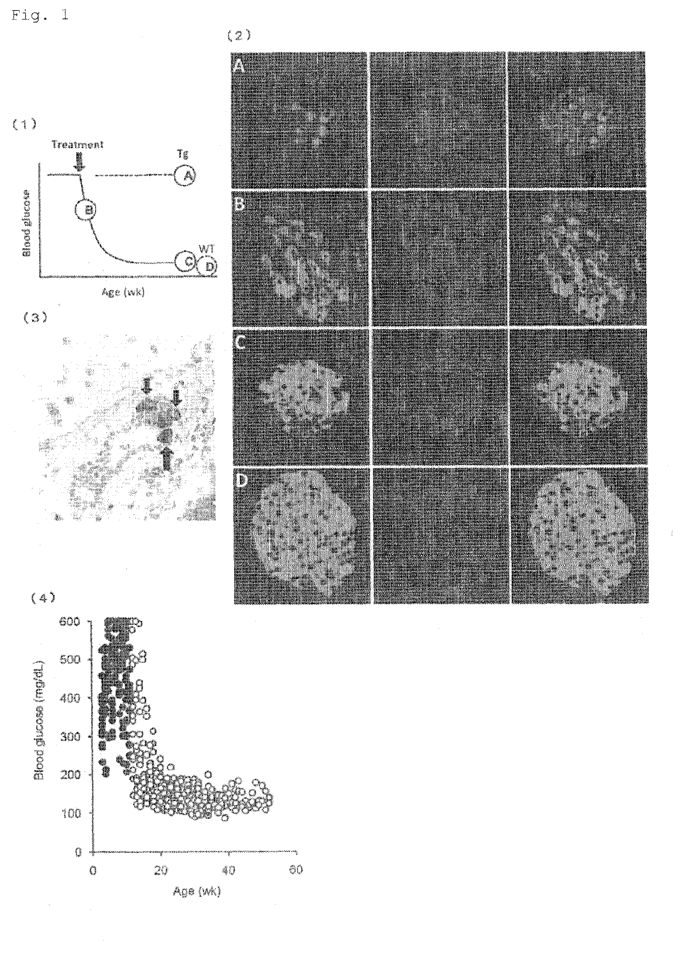

Fig. 1 shows results from Experimental Example 1. Shown in (1) are: change (indicated as a solid line in the figure) in blood glucose level of an estradiol administration group (Tx+E group); change (indicated as a dotted line in the figure) in blood glucose level in a control group (Tg group); and blood glucose level (indicated as D in the figure) of wild-type mice (WT group). Shown in (2) are the presence and state of insulin-producing cells (stained in green in the figure) and glucagon-producing cells in pancreatic islets of each of the mice (A: Tg group (48-55 weeks of age), B: Tx+E group (24 weeks of age) at 1 week after estradiol administration, C: Tx+E group (48-55 weeks of age) at 36-43 weeks after estradiol administration, and D: WT group (48-55 weeks of age)). In (2), the insulin-producing cells are stained in green (left column), and the glucagon-producing cells are stained in red (central column). The right column in (2) shows a superimposition of the insulin-producing cells and the glucagon-producing cells. (3) is an image showing a result of immunostaining of pancreas at a time point of B (1 week after estradiol administration) for the estradiol administration group (Tx+E group) using an anti-insulin antibody; and cells, which are stained in brown and are indicated by arrows, are insulin-producing cells developing in the pancreatic duct epithelium. Shown in (4) are blood glucose levels of each Tg23 mouse before (indicated by filled circle in the figure) and after (indicated by open circle in the figure) administration of 17β-estradiol (E) (in the figure, each of the filled circle and open circle represents an individual mouse; however, mice having the same numerical value are represented by a single open circle, regardless of the number of such mince.).

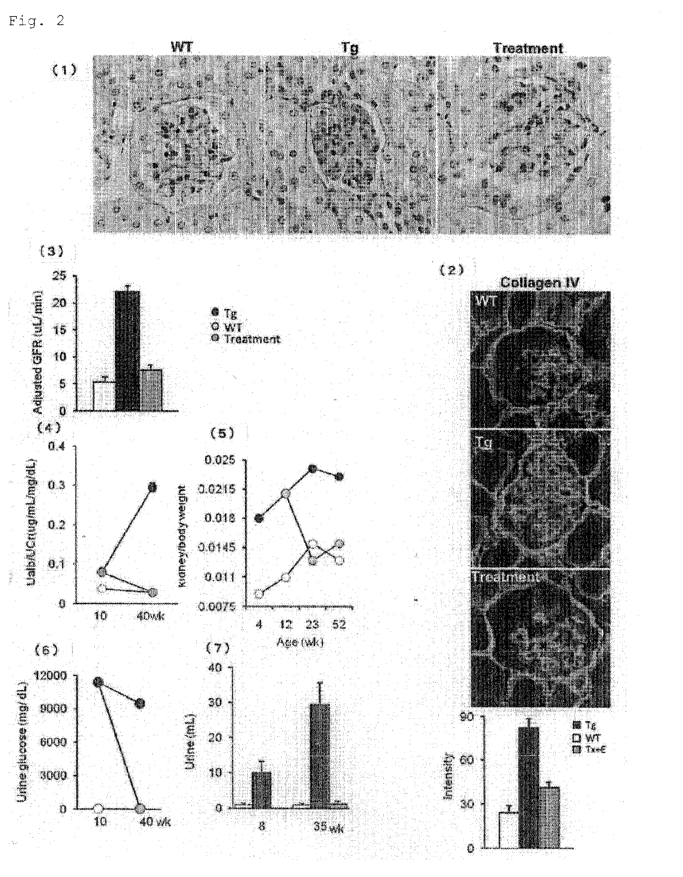

Fig. 2 shows results from Experimental Example 2. (1) shows results of PAS staining of glomerular tissues of mice in each group (Tg group, Tx+E (Treatment) group, WT). (2) shows a result of immunostaining of glomerular tissues of each of the mice (WT, Tg group, Tx+E (Treatment) group) using an anti-collagen IV antibody. Expression intensities of collagen IV in the glomeruli are semiquantified, and the intensities are shown at the bottom of (2) as average pixel intensities. (3) shows glomerular filtration rates (μL/min) for the mice in each of the groups (Tg group, Tx+E (Treatment) group, WT); (4) shows urinary albumin amount / uric acid clearance (Ualb/Cr) (μg/mg/dL) for the mice in each of the groups; (5) shows change (week) in kidney weights (g) for the mice in each of the groups; (6) shows urinary glucose levels (mg/dL) for the mice in each of the groups; and (7) shows urine volume (mL) per day for each of the mice.

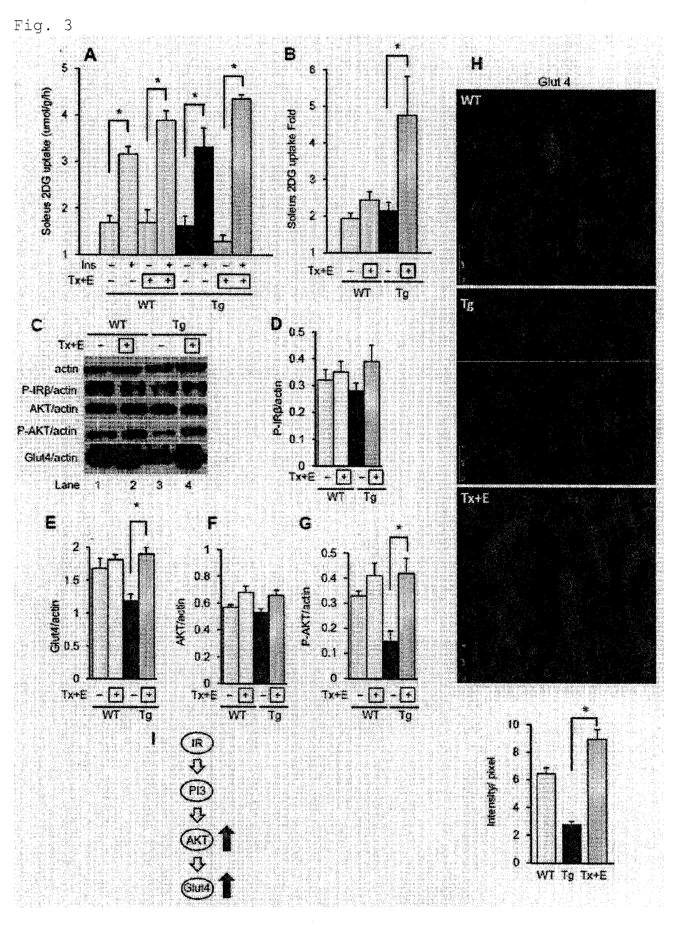

Fig. 3 shows results from Experimental Example 3. B shows relative ratios (folds) of an amount of glucose intake in the presence of insulin with respect to an amount of glucose intake in the absence of insulin, for WT mice in Ct group (WT: -) and in Tx+E group (WT: +) , and for Tg mice in Ct group (Tg: -) and in Tx+E group (Tg: +). C is a western blot image for measuring intramuscular expression levels of actin, P-IRp, AKT, P-AKT, and Glut4 (represented as relative ratios with respect to an expression level of actin) for WT mice in Ct group (WT: -) and in Tx+E group (WT: +), and for Tg mice in Ct group (Tg: -) and in Tx+E group (Tg: +). D, F, G, and E are results comparing expression intensities of each of the molecules (P-IRβ, ANT, P-AKT, and Glut4) among each of the mice groups. H shows images for observing the existence of Glut4 with immunostaining performed on muscular tissues using an anti-Glut4 antibody for WT mice in Ct group (WT), and for Tg mice in Ct group (Tg) and in Tx+E group (Tx+E); and, located below the images is a result of semiquantifying Glut4 expression intensity, which is represented as an intensity with respect to a pixel, i.e., "Intensity/Pixel." I is a schematic diagram showing signal transduction involved in glucose intake. A shows a result obtained by: incubating, for 10 minutes at 30°C in KRB buffer containing 1 mM 2-deoxy-D-[$^3$H]glucose (1.5 βCi-/ml) and 7 mM D-[$^{14}$C]mannitol (0.3 βCi/ml), muscles (soleus muscles) of each of the mice (WT and Tg mice) in an estradiol administration group (represented as "Tx+E: +" in Fig. 3A) and in a control group (represented as "Tx+E: -" in Fig. 3A), in presence of insulin (represented as "Ins: +" in Fig. 3A) and in absence of insulin (represented as "Ins: -" in Fig. 3A); and measuring the amount (βmol/g/h) of glucose intake of the muscles.

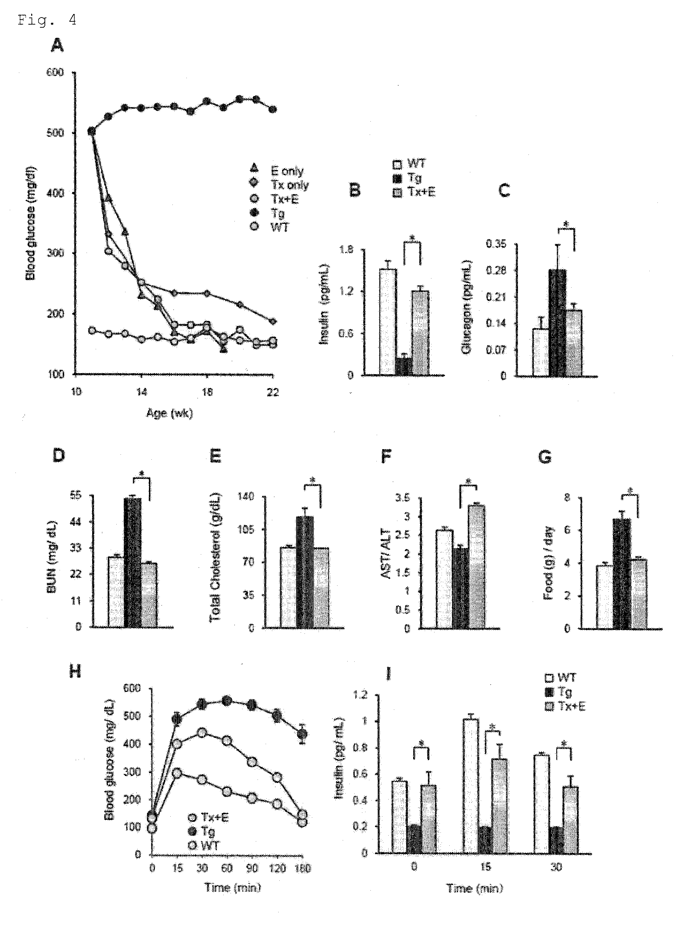

Fig. 4 shows results from Experimental Example 4. A shows changes in blood glucose levels (mg/dl) over time after administering estradiol (or a pellet that does not include estradiol) to mice in E-only group, Tx-only group, Tx+E group, and Tg group, and to wild-type mice (WT). In addition, B, C, D, E, F, and G respectively show measurements performed with Tx+E group, Tg group, and wild-type mice (MT) for serum insulin concentrations (pg/mL), serum glucagon concentrations (pg/mL), blood urea nitrogen (BUN) concentrations (mg/dL), total serum cholesterol levels (g/dL), aspartate aminotransferase (AST) / alanine aminotransferase (ALT), and food consumption per day (g/day). H and I respectively show changes over time in blood glucose levels and serum insulin concentrations measured after conducting oral glucose tolerance test on Tx+E group, Tg group, and wild-type (WT) mice.

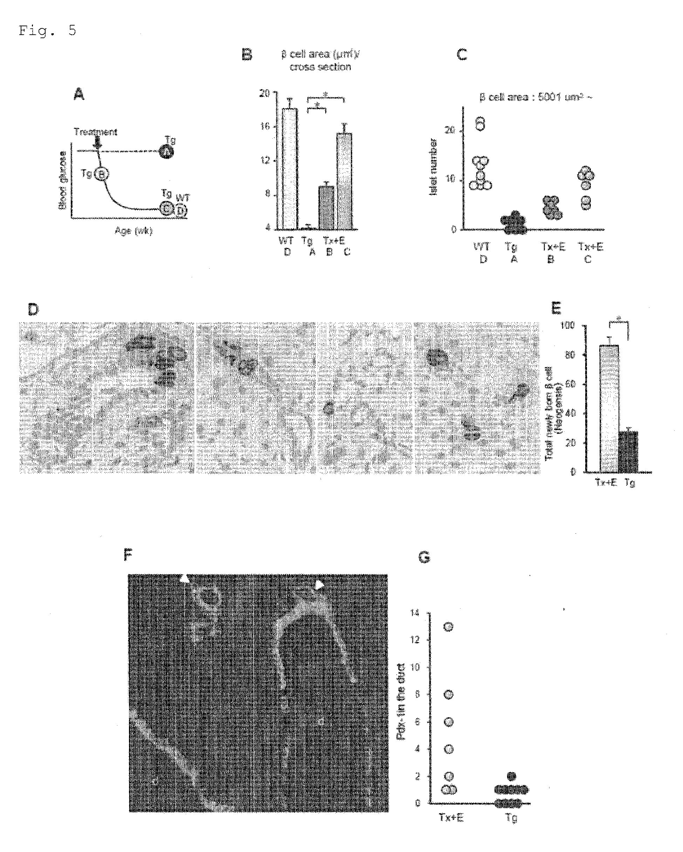

Fig. 5 shows results from Experimental Example 5. Shown in A are: change (indicated by a solid line in the figure) in blood glucose level of Tx+E group; change (indicated as a dotted line in the figure) in blood glucose level of Tg group; and blood glucose level (indicated as D in the figure) of wild-type mice (WT group) . In addition, B and C respectively show area (βm$^2$) of β-cells on a single section of pancreatic tissues, and the number of pancreatic islets whose area of β-cells is 5001 μm$^2$ or larger, for each of the mice at the time of blood glucose level measurement (A: Tg group (55 weeks of age), B: Tx+E group (24 weeks of age) at 1 week after estradiol administration, C: Tx+E group (55 weeks of age) at 43 weeks after estradiol administration, and D: WT group (55 weeks of age)). All of the images in D show results of immunostaining of pancreases at a time point of B (1 week after estradiol administration) for Tx+E group using the anti-insulin antibody; and insulin-producing cells in the pancreatic duct epithelial cells are shown in brown. E shows a result of comparing the number of insulin-producing cells that were newly generated in the pancreatic duct epithelial cells of Tx+E group and Tg group (both 23-24 weeks of age). 5F shows a result of observing expression of Pdx-1 (Pancreas duodenum homeobox 1) by immunostaining the pancreases of Tx+E group and Tg group (both 23-24 weeks of age) using an anti-Pdx-1 antibody; and G shows a result of comparing the numbers of Pyx-1 in the pancreatic duct epithelial cells of Tx+E group and Tg group (both 23-24 weeks of age).

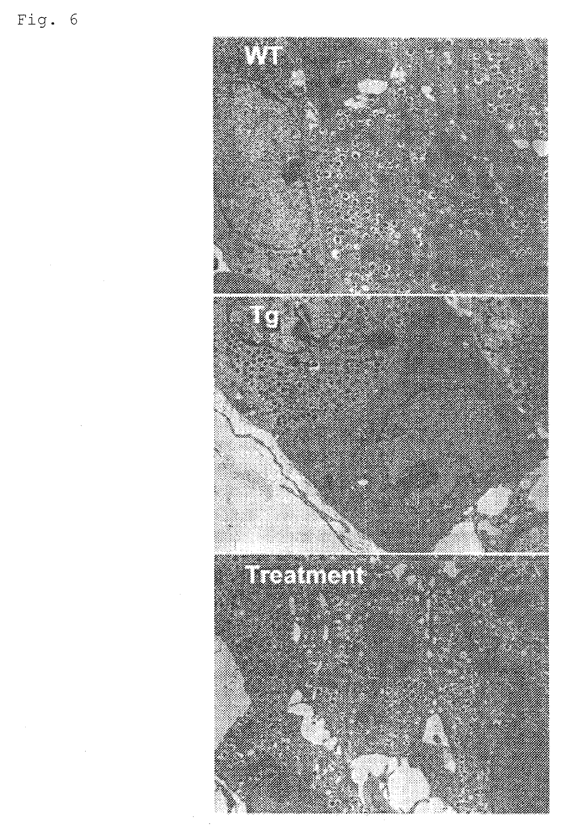

Fig. 6 shows a result from Experimental Example 5 of preparing sections of pancreatic tissues of mice in Tx+E group (Treatment) and in Tg group, and wild-type mice (WT) (all 48 weeks of age), and observing the sections under an electron microscope.

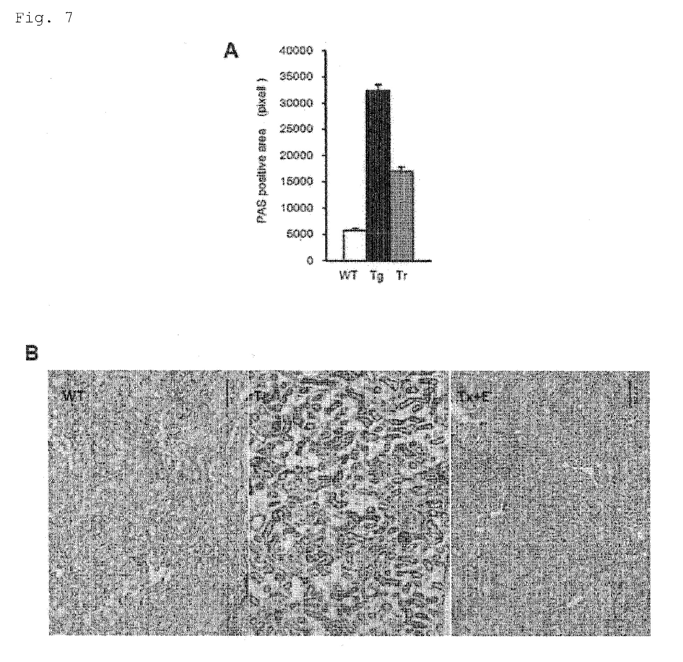

Fig. 7 shows (A) a result of comparing PAS-positive areas (pixels) in the glomeruli, and (B) a result of PAS staining performed on cells/tissues of renal tubules, for mice in Tx+E group (Treatment) and in Tg group, and wild-type mice (WT) (all 48 weeks of age) in Experimental Example 5.

Description of Embodiments

As described herein, "estrogenic compound" includes compounds that have ovarian hormonal effects similar to estrogens, and includes both steroidal estrogens and synthetic estrogens. The steroidal estrogens include: estradiol, esters of estradiol (including, for example, a benzoic ester of estradiol, a propionate ester of estradiol, a valerate ester of estradiol, and the like), oestrone, estriol, esters of estriol (including, for example, esters such as an acetic ester, a propionate ester, and a benzoin ester of estriol), equilin, ethinylestradiol, conjugated estrogens (a conjugated preparation of oestrone and equilin), and the like. Furthermore, the synthetic estrogens include stilbestrol, hexestrol, and the like. With regard to those described above, a single type may be used by itself, or an arbitrary combination of two or more types may be used. Preferably, estradiol or an ester thereof is used.

Insulin-producing cells induced by the estrogenic compound refer to insulin-secreting cells that occupy 70% to 80% of a pancreatic Langerhans islet. The insulin-producing cells, whose β-granules that store insulin can be observed with an electron microscope, secrete insulin through exocytosis caused by secretory stimulation from glucose. With the present invention, insulin-producing cells are induced in pancreatic ducts by the estrogenic compound. Although insulin-producing cells do not originally exist in the pancreatic ducts, some of the cells in the pancreatic ducts are changed into insulin-producing cells by an effect of the estrogenic compound. When the function and number of pancreatic cells are reduced, the production amount of insulin decreases. However, with the present invention, even for patients whose β-cell functions are completely lost and who need insulin injections, insulin-producing cells can be induced in pancreatic ducts; and, due to insulin release from the pancreatic ducts, the number of insulin injections (or administration dose) can be reduced, or the insulin injections become unnecessary.

There are two types of "diabetes": type 1 diabetes, whose symptoms develop based on the dying of β-cells that secrete insulin in the Langerhans islets in the pancreas; and type 2 diabetes, whose symptoms develop due to a decrease in insulin secretion and a reduced sensitivity to insulin. "Diabetes" as a target of the present invention includes both of these types. In particular, with the present invention, considering the fact that the insulin-producing cells induced in the pancreatic ducts by the estrogenic compound can take over the functions of the β-cells, the present invention can be applied more effectively to patients with type 1 diabetes.

"Diabetes complication" includes: acute complications (diabetic comas (ketotic coma, nonketotic hyperosmolar coma, lactic acidosis, and hypoglycemic coma), and acute infections); and chronic complications (microangiopathies (diabetic retinopathy, diabetic nephropathy, and diabetic neuropathy), macroangiopathies (cerebrovascular disorder, ischemic cardiopathy, and diabetic gangrene), and others (hyperlipidemia, chronic infections, cholelithiasis, cataract, and the like)). diabetes complication" as a target of the present invention includes all of those described above; however, the diabetes complication is preferably a chronic complication. The diabetes complication is more preferably a microangiopathy such as diabetic retinapathy, diabetic nephropathy, diabetic neuropathy, and the like; and is particularly preferably diabetic nephropathy.

"Diabetic nephropathy" can be characterized in diabetics by proteinuria, abnormal kidney tissue alterations such as glomerulosclerosis, interstitial alterations, and vascular lesions; or reduction in glomerular filtration rate. When diabetic nephropathy symptoms develop, it has been difficult to completely recover from this disease, and delaying the progress thereof has been the only option. However, with the present invention, clear improvements are observed in organic alterations such as sclerosis of the glomerulus, interstitial alterations, and vascular lesions, and it is possible to completely recovery from diabetic nephropathy. When diabetic nephropathy progresses, kidney dialysis that requires large medical expenses becomes necessary. Thus, to be able to treat diabetic nephropathy is also extremely important from the point of view of health care economics.

With the present invention, it has been found that the estrogenic compound has an enhancing effect of glucose intake of muscles. For mammals including human, muscles occupy a large proportion of the body. Therefore, enhancing intake of glucose by muscles results in immediate lowering of the blood glucose level.

With the therapeutic agent for diabetes or a diabetes complication, the insulin-producing cell inducer, and the glucose intake enhancer of the present invention, the estrogenic compound can be administered, to a subject (manual, preferably human) in need of the compound, in an effective dose in the form of a pharmaceutical composition that also includes a pharmaceutically acceptable carrier.

Furthermore, the estrogenic compound of the present invention can be combined and administered together with another therapeutic agent for diabetes.

The "effective dose" can be set in accordance with the symptoms (diabetes symptoms or diabetes complications, reduction in the number and function of insulin-producing cells, and hyperglycemia) that should be treated in a subject (mammal, preferably human (patient)) in need. The "effective dose" refers to an amount necessary for relieving or remitting these symptoms.

The specific administration dose differs depending on the type and effect of the estrogenic compound, age, body weight, sex, and severity of the symptoms of the subject; and the like. However, for adults, the dose is generally about 0.01 mg to 100 mg per day, and preferably about 0.1 mg to 50 mg per day. The dose is administered once a day, or is divided and provided as two to four administrations per day.

An active ingredient of the pharmaceutical composition (the therapeutic agent for diabetes or the diabetes complication, the insulin-producing cell inducer, and the glucose intake enhancer) of the present invention is the estrogenic compound. A mode for administering the pharmaceutical composition is not particularly limited, and can be chosen as appropriate in accordance with the therapeutic goal. For example, administration modes such as oral solid preparations, oral liquid preparations, nasally administered agents, transpulmonary administered agents, injectable agents, intravenous agents, suppositories, external preparations, and patches can be used. These administration modes can be produced by blending in a pharmaceutically acceptable carrier, and producing a preparation using methods that are known and commonly used by those skilled in the art.

When preparing an oral solid preparation, the estrogenic compound may be admixed with an excipient, and, optionally, with a binder, a disintegrant, a lubricant, a coloring agent, a corrigent, an odor-masking agent, or the like. Then, the mixture may be made into a form of a tablet, a granule, powder, a capsule, or the like by using a method commonly used in the art. As such additives, those commonly used in the art may be used. Exemplary excipients include lactose, sodium chloride, glucose, starch, microcrystalline cellulose, and silicic acid. Exemplary binders include water, ethanol, propanol, simple syrup, a gelatin solution, hydroxypropyl cellulose, methyl cellulose, ethyl cellulose, shellac, calcium phosphate, and polyvinylpyrrolidone. Exemplary disintegrants include powdered agar, sodium bicarbonate, sodium lauryl sulfate, and stearic monoglyceride. Exemplary lubricants include purified talc, stearate, borax, and polyethylene glycol. Exemplary coloring agents include β-carotene, yellow ferric oxide, and caramel. Exemplary corrigents include white soft sugar and bitter orange peel.

When preparing an oral liquid preparation, the estrogenic compound may be admixed with a corrigent, a buffer, a stabilizing agent, a preservative, or the like. Then, the mixture may be made into a form of a liquid preparation, a syrup, an elixir, or the like by a method commonly used in the art. As such additives, those commonly used in the art may be used. Exemplary corrigents include white soft sugar, exemplary buffers include sodium citrate, exemplary stabilizing agents include tragacanth, and exemplary preservatives include p-hydroxybenzoate ester.

When preparing an injectable agent, the estrogenic compound may be admixed with a pH regulator, a stabilizing agent, a tonicity adjusting agent, or the like. Then, using a method commonly used in the art, the mixture may be made into an agent that can be injected subcutaneously, intramuscularly, or intravenously. As such additives, those commonly used in the art may be used. Exemplary pH regulators include sodium phosphate, exemplary stabilizing agents include sodium pyrosulfite, and exemplary tonicity adjusting agents include sodium chloride.

When preparing a suppository, the estrogenic compound may be admixed with a carrier, a surfactant, or the like; and the mixture may be made into a suppository using a method commonly used in the art. As such additives, those commonly used in the art may be used. Exemplary carriers include polyethylene glycol and hard fat, and exemplary surfactants include Polysorbate 80.

When preparing an external preparation, the estrogenic compound may be admixed with a base, a water-soluble polymer, a solvent, a surfactant, a preservative, or the like; and then the mixture may be made into the form of a liquid agent, a cream, a gel, an ointment, a plaster, or the like using a method commonly used in the art. Exemplary bases include liquid paraffin, white petroleum jelly, purified lanolin, or the like. Exemplary water soluble polymers include carboxyvinyl polymers. Exemplary solvents include glycerol and water. Exemplary surfactants include polyoxyethylene fatty acid ester. Exemplary preservatives include p-hydroxybenzoate ester.

When preparing a patch, the estrogenic compound may be admixed with an adhesive, a solvent, a cross linking agent, a surfactant, or the like; and the mixture may be made into a water-based patch, a plaster patch, or the like using a method commonly used in the art. As such additives, those commonly used in the art may be used. Exemplary adhesives include polyacrylic acid partially neutralized products, sodium polyacrylate, 2-ethylhexyl polyacrylate, and styrene-isoprene-styrene block copolymers. Exemplary solvents include glycerol and water. Exemplary cross linking agents include dihydraxyaluminum amino acetate and dried aluminium hydroxide gels. Exemplary surfactants include polyoxyethylene fatty acid ester.

Examples

In the following, the present invention will be described in detail using experimental examples. However, the present invention is not limited to the examples described below.

Various measurements shown in each of the experimental examples were measured in accordance with the below-described methods.

<u>(A) Experimental methods</u>

<u>(1) Blood glucose level (blood sugar level)</u>

Blood glucose levels were measured by an enzyme-electrode method with ONE TOUCH UltraVue (Johnson & Johnson K.K.) on whole blood taken from tail veins of subject animals.

<u>(2) HbA1c</u>

HbA1c was measured in whole blood taken from tail veins of subject animals using DCA2000 analyzer (Bayer Medical).

<u>(3) Other blood parameters</u>

Immediately before collecting organs, blood parameters were measured using various methods and measurement kits described below in blood collected from the hearts of animals anesthetized with nembutal.

(a) Creatinine: High performance liquid chromatography (HPLC),
(b) Albumin: BCG method,
(c) Blood urea nitrogen (BUN): Urease UV method,
(d) Aspartate aminotransferase (AST) and alanine aminotransferase (ALT): JSCC standardization method,
(e) Serum insulin concentration: Enzyme-linked immunosorbent assay kit (Morinaga Institute of Biological Science),
(f) Serum glucagon concentration: YK142 Mouse GLP-2 EIA kit (Yanaihara Institute Inc.),
(g) Total serum cholesterol level: Enzymatic assay reagent (Wako Pure Chemical Industries),
(h) Serum estradiol concentration: Radioimmunoassay reagent (DPC-TKTTI; Diagnostic Products Co.), and
(i) Serum testosterone concentration: Radioimmunoassay reagent (DPC-KE2D1; Diagnostic Products Co.).

<u>(4) Urinary parameters</u>

Urinary parameters were measured using various methods and measurement kits described below.

(a) Albumin concentration: Albuwellkit (Exocell),
(b) Creatinine: High-performance liquid chromatography (HPLC), and
(c) Creatinine clearance (Ccr): calculated by the next formula.

[Math.1]

$$Ccr = \text{urine creatinine (mg/dL)} \times \text{urine volume (μL/min)} / \text{serum creatinine (mg/dL)} / \text{body weight (g)}.$$

<u>(5) Tissue staining (Immunostaining)</u>

Pancreases were fixed in 10% formalin, embedded in paraffin, and sliced into sections having a thickness of 2 μm. The sections were immunostained with overnight incubation at 4°C in dilution solutions containing various primary antibodies described below.

Primary antibodies: Anti-insulin antibody (dilution rate 1:500; DAKO), anti-glucagon antibody (dilution rate 1:500; Linco Research Inc.), anti-DBA antibody (dilution rate 1:300; Vector Laboratories), anti-Pdx-1 antibody (dilution rate 1:1000; kindly provided by Chris V.E. Wright, Vanderbilt University), anti-collagen type IV antibody (dilution rate 1:250; PROGEN Biotechnik), and anti-Glut 4 antibody (dilution rate 1:1000; Chemicon).

The primary antibodies were detected by immunofluorescence labeling using secondary antibodies conjugated with Alexa 488 or Alexa 568 (1:200; Molecular Probes); third antibody conjugated with biotin-labeled secondary antibodies and streptavidin Alexa 488 or Alexa 568 (1:800; Vector Laboratories); or by an immunoperoxidase staining method using biotin-labeled secondary antibodies. Staining was visualized with diaminobenzidine. The sections were counterstained with hematoxylin. Images were captured on a BZ9000 microscope (Keyence), or in the confocal mode on an LSM 510 META confocal microscope (Carl Zeiss). Immunofluorescence intensity was measured in random fields (n = 30 / section) using LSM Zeiss software, and the result was expressed as average pixel intensity.

(6) Quantification of the numbers and areas of β-cells, α- cells, and pancreatic islets on pancreas section

All pancreatic islets, glucagon positive cells, and insulin-positive cells existing on a single pancreas section were photographed on a Keyence Biorevo microscope, and five to seven sections corresponding to areas that are at least 150 μm apart from one another were evaluated for a single pancreas (a single group includes five to eight males and six to nine females). Areas of β-cells and pancreatic islets were measured for each of the sections using Image Joint and Dynamic Cell Count software (Keyence).

(7) Oral glucose tolerance test (OGTT)

After a 16-hour fast, mice were administered with glucose (1 g / kg of body weight) by gavage. Blood samples were collected from the tail vein at 0, 15, 30, 60, 90, and 120 minutes after the oral administration, and blood glucose levels were measured. In addition, blood samples were collected from the tail vein in a similar manner at 0, 15, and 30 minutes after the oral administration, and serum insulin concentrations were measured.

(8) Measurement of food consumption and water consumption

Subject mice were individually housed in metabolic cages for four days to assess food consumption (g / 24 h) and water consumption (ml / 24 h) . During measurement, food and water were supplied to the mice *ad libitum.* Measurements were repeated three times for each group.

(9) Electron microscopy

In accordance with a method commonly used in the art, organs (pancreas and kidney) collected from the mice were fixed with 2% glutaraldehyde in 0.05 mol/L phosphate buffer, dehydrated in a graded ethanol series, and embedded in epoxy resin. The organs were placed in a Reichert-Nissei Ultracuts microtome (Leica Microsystems, Wetzlar, Germany), and cut with a glass knife to prepare sections. The prepared sections were double-stained with uranyl acetate and lead citrate, and observed with a Hitachi H7100 electron microscope (Hitachi, Ltd., Tokyo, Japan).

(10) Measurements of glomerular surface area, PAS-positive area, and glomerular number

[0080]　50 glomeruli were analyzed for each of the mice. The total area of the glomeruli was measured in PAS-stained sections using Image-Pro Plus (Media Cybernetics). The PAS-positive area fraction was calculated as the ratio of PAS-positive area to glomerular sectional surface area. Glomerular number was measured by counting all of the glomeruli per mid-transverse section of left kidney for each of the mice.

(11) Muscle incubation *in vitro*

Mice were sacrificed, and extensor digitorum longus (EDL) muscles and soleus muscles were quickie removed and processed. Both ends of each of the muscles were tied with suture (silk 4-0), and mounted on an incubation apparatus. First, the muscles were preincubated for 20 minutes in 6 ml of Krebs-Ringer bicarbonate (KRB) buffer (117 mM NaCl, 4.7 mM KCl, 2.5 mM $CaCl_2$, 1.2 mM $KH_2PO_4$, 1.2 mM $MgSO_4$, 24.6 mM $NaHCO_3$, pH 7.5) containing 2 mM pyruvate. Then, the muscles were incubated in KRB buffer in presence or absence of insulin.

(12) Measurement of intramuscular glucose transport *in vitro*

Immediately after muscle incubation, the muscles were transferred to KERB buffer containing 1 mM 2-deoxy-D-[$^3$H] glucose (1.5 μCi/ml) and 7 mM D-[$^{14}$C]mannitol (0.3 μCi/ml) (PerkinElmer Life Sciences) at 30°C, and incubated again for 10 minutes. When using insulin, a buffer containing insulin was used for incubation in a similar manner.
Glucose transport was terminated by dipping the muscles in KRB buffer containing 80 μmol/L cytochalasin B at 4°C, and then the muscles were frozen in liquid nitrogen. The muscles were weighed, and digested by incubating in 250 μl of 1 M NaOH at 8C°C for 10 minutes. Digests were neutralized with 250 μl of 1 M HCl. Then, particulates were precipitated by centrifugation at 13,000
× g for 5 minutes. Radioactivity in aliquots of the digested muscle was determined by liquid scintillation counting for dual labels, and extracellular and intracellular spaces were calculated.

(13) Measurement of intramuscular glycogen concentration

Muscles were treated in 30% KOH and 5% $Na_2SO_4$ at 70°C for 15 minutes to be dissolved. By mixing a three times-volume of absolute alcohol thereinto, glycogen was precipitated, and the mixture was stored overnight at -20°C. Precipitates were collected by centrifugation at 13,000 g for 5 minutes. Glycogen was processed in 6N $H_2SO_4$ at 100°C for 45 minutes to be hydrolyzed, and then, the mixture was cooled. The obtained sample was neutralized in 1N NaOH, and glucose level was measured using glucose (HK) reagent (Sigma Chemical).

(14) Western blot assay

Mice were sacrifice, and the EDL, tibialis anterior, and gastrocnemius muscles were removed and snap-frozen in liquid nitrogen. Pulverized muscles were homogenized with a Polytron in an ice-cold radioimmune precipitation buffer (50 mM Tris-HCl (pH 7.4), 150 mM NaCl, 1% Triton X-100, 1% sodium deoxycholate, 0.1% SDS, 22 mM EDTA) containing Protease Inhibitor Cocktail (Sigma) and Phosphatase Inhibitor Cocktail (stigma). After removing insoluble debris by centrifugation, supernatants (40 $\mu$g) were processed using 12.5% SDS-PAGE, and transferred to nitrocellulose membranes (Millipore Corp.). The membranes were blocked with 5% skim milk in phosphate-buffered saline, and then incubated overnight at 4°C with a primary antibody of anti-Akt antibody (1:1000; Cell Signaling Technology), anti-Ser473-phosphorylated Akt antibody (1:2000; Cell Signaling Technology), anti-Glut4 antibody (1:1000; Chemicon), anti-actin antibody (1:3000; Sigma), or anti-Tyr972-phosphorylated IRβ antibody (1:1000; Calbiochem). Then, the obtained membranes were incubated for one hour at room temperature with a horseradish peroxidase-linked anti-rabbit IgG antibody (used in a proportion of 1:2000 for each of the primary antibodies other than the anti-actin antibody, and used in a proportion of 1:5000 for the anti-actin antibody; Molecular Robe) . Each of these steps was followed by three washes for 10 minutes in 5% Tween 20/phosphate-buffered saline. Detection was performed as described in the ECL protocol (Amersham Pharmacia Bintech). Antibodies were removed from the membranes by processing the membranes for 30 minutes in stripping buffer (100 mM 2-mercaptoethanol, 2% SDS, 62.5 mM Tris-HCl, pH 6.7) (55°C). Then, a second immunoprobing was performed to detect actin as an internal control. Immunoblotting experiments were repeated three times or more.

Protein levels were quantified using NIH Image. Signal intensity was obtained as PICT files by a slide scanner.

(B) Veneration of subject animals

Generation of animal model for diabetes (ICERIγ transgenic mouse)

ICERIγ transgenic mice (male) were generated from a pure line C57BL/6J (Japan SLC, Inc.) in accordance with the method described in Example 1 of Patent Literature 1 (WO2004/072282). From 62 mice that were generated, 3 transgenic mice (Tg7, Tg12, and Tg23 mice: hereinafter collectively referred to as "Tg mice") were obtained. The copy numbers of ICERIγ gene introduced in these mice ((Tg7, Tg12, and Tg23 mice) were 4, 4, and 6, respectively. In these Tg mice, there is a clear diabetes onset mechanism, in which, by overexpressing ICERIγ in β-cellsr insulin synthesis and growth of the β-cells are suppressed; and blood glucose level becomes high, and severe diabetes symptoms develop due to insulin secretion failure. Thus, the Tg mice (Tg12 and Tg23 mice) were used as diabetes model mice in the following experiments.

In addition, non-transgenic wild-type mice (WT mice) were used for control experiments.

The mice described above were housed in microisolator cages at a room temperature of $24 \pm 2$°C, in a humidity (relative humidity) of $50 \pm 10$%, and under a 12-hour light/dark cycle. Furthermore, water and food (standard rodent diet (CE-2; CLEA Japan, Inc.) were supplied *ad libitum.* All of the mice were handled in accordance with the Guidelines for Animal Experiments of Kyushu University.

experimental Example 1: Effect of estradiol administration

Tg23 mice (11 weeks of age) were divided into two groups: an estradiol administration group (n = 24), and a control group (n = 57). For the estradiol administration group, testectomy (Tx) was performed, and a single pellet of 17β-estradiol (E) (contained amount of E is 0.72 mg / pellet; 90-day time release; maximum release amount of estradiol per day is 0.008 mag; the same applies for the following experimental examples) was subcutaneously administered (implanted) at the nape of the neck. For the control group, sham surgery of testectomy was performed, and a single pellet (Innovative Research of America) that consists of a vehicle and does not include 17β-estradiol was subcutaneously administered at the nape of the neck. Then, body weight and blood glucose levels of the Tg mice and wild-type mice (WT) (n = 81) were monitored every week, and the mice were dissected after 43 weeks.

Shown in Fig. 1(1) are: change (indicated by a solid line in the figure) in blood glucose level of the estradiol administration group (Tx+E group); change (indicated as a dotted line in the figure) in blood glucose level of the control group (Tg group); and blood glucose level (indicated as D in the figure) of wild-type mice (WT group) . Shown in Fig. 1 (2) are the presence and state of insulin-producing cells (stained in green in the figure) and glucagon-producing cells in pancreatic islets of each of the mice at the time of blood glucose level measurements (A: Tg group (48 weeks of age), B: Tx+E group (24 weeks of age) at 1 week after estradiol administration, C: Tx+E group (48 weeks of age) at 36 weeks after estradiol administration, and D: WT group (48 weeks of age)) . In Fig. 1(2), the insulin-producing cells are stained in green (left column), and the glucagon-producing cells are stained in red (central column). The right column in Fig. 1 (2) shows a superimposition of the insulin-producing cells and the glucagon-producing cells. Fig. 1(3) is an image showing a result of immunostaining of pancreas at a time point of B (1 week after estradiol administration) for the estradiol administration group (Tx+E group) using the anti-insalin antibody; and cells, which are stained in brown and are indicated by arrows, are insulin-producing cells. Shown in Fig. 1(4) are blood glucose levels of each of the Tg23 mice before (indicated by filled circle in the figure) and after (indicated by open circle in the figure) administration of 17β-estradiol (E) (in the figure, each of the filled circle and open circle represents an individual mouse; however, mice having the same numerical value are represented by a single open circle, regardless of the number of such mice).

This result showed that, in the estradiol administration group (Tx+E group) obtained by administering estradiol to diabetes model mice (Tg mice), the blood glucose levels were lowered to the same levels of normal mice (wild-type mice), and insulin-producing cells were induced in pancreatic duct epithelium cells of the pancreas. With this, it was confirmed that administering estradiol to subject animals of diabetes causes rapid neogenesis and growth of β-cells and normalization of blood glucose level. It should be noted that the lowered blood glucose levels were maintained until death.

Experimental Example 2: Effect of estradiol on the second stage (early nephropathia stage) to the third stage (intermediate stage) of diabetic nephropathy

For Tg23 mice, at 20-35 weeks of age, which is when reduction in renal functions and renal lesions are observed, a single pellet of 17β-estradiol was subcutaneously administered, and the effect of estradiol was investigated. The above-described weeks-of-age for Tg mice corresponds to the second stage (early nephropathia stage) to the third stage (intermediate stage) of diabetic nephropathy; and it is said that, usually, renal lesions do not return to normal after this stage, even if blood glucose level returns to normal.

Specifically, Tg23 mice (24 weeks of age) were divided into two groups: an estradiol administration group, and a control group. For the estradiol administration group (Tx+E group, also referred to as Treatment group), testectomy (Tx) was performed, and a single pellet of 17β-estradiol (E) was subcutaneously administered at the nape of the neck. For the control group (Tg group), sham surgery of testectomy (Tx) was performed, and a single pellet that consists of a vehicle and does not include 17β-estradiol was subcutaneously administered at the nape of the neck. Then, measurements were performed in the Tx+E group, Tg group, and wild-type mice (WT), for glomerular filtration rate (GFR) ($\mu$L/min), urinary albumin amount / uric acid clearance (Ualb/Cr) ($\mu$g/mg/dL), kidney weight (g), urinary glucose level (mg/dL), and urine volume (mL) .

PAS staining, and immunostaining using the anti-collagen type IV antibody were performed on glomerular tissues from each of the mice (48 weeks of age), and the results of the staining are shown respectively in Fig. 2(1) and Fig. 2(2) . Shown at the bottom of Fig. 2(2) is the result of semiquantifying expression intensity of collagen IV in the glomeruli using LSM Zeiss software. The expression intensity was measured in random fields (n = 30 / section) of the glomerulus, and the result was expressed as average pixel intensity.

Fig. 2(3) shows glomerular filtration rates ($\mu$L/min) for the mice in each of the groups (Tg group, WT, Tx+E group (Treatment)) (40 weeks of age); Fig. 2(4) shows urinary albumin amount / uric acid clearance (Ualb/Cr) ($\mu$g/mg/dL) for the mice in each of the groups (10 weeks of age, 40 weeks of age); FIG. 2(5) shows changes (weeks) in kidney weight (g) for the mice in each of the groups; Fig. 2(6) shows urinary glucose level (mg/dL) for the mice in each of the groups (10 weeks of age, 40 weeks of age), and Fig. 2(7) shows urine volume (mL) per day for each of the mice (8 weeks of age, 35 weeks of age).

From the results in Fig. 2(1) and (2), it was confirmed that, in the glomeruli of the Tg mice (Treatment mice) administered with estradiol, sclerosis was alleviated to a degree not different from wild-type mice (WT). In addition, from the results in Fig. 2(3) to (7), it was confirmed that, even for conditions of the second stage of diabetic nephropathy (early nephropathia stage), administering estradiol dramatically improves renal functions and renal tissues. On the other hand, in Tg mice that did not receive estradiol administration, the sclerosis of the glomeruli further progressed from the early nephropathia stage during the course of time (cf. Fig. 2(1) and (2)), and aggravation in the diabetic nephropathy was observed (cf. Fig. 2(3), (4), and (7))

Heretofore, it has been considered that recovering (treatment) from diabetic nephropathy becomes extremely difficult after passing the second stage (early nephropathia stage), which is a turning point. The above results deserve attention because they show that administration of estradiol caused dramatic improvement to renal functions and renal tissues, even after passing the second stage (early nephropathia stage).

Experimental Example 3

Wild-type mice (male) and Tg23 mice (male) were each divided into two groups (an estradiol administration group (n = 6 to 10) and a control group (n = 6 to 10)). It should be noted that sugar intake had been deteriorated in Tg23 mice (11 weeks of age) due to hyperglycemia / low insulin.

For each of the mice (WT and Tg mice) in the estradiol administration group (Tx-E group), testectomy (Tx) was performed, and a single pellet of 17β-estradiol (E) was subcutaneously administered at the nape of the neck at 11 weeks of age. In addition, for each of the mice (WT and Tg mice) in the control group (Ct group), sham surgery of testectomy (Tx) was performed, and a single pellet (Innovative Research of America) that consists of a vehicle and does not include 17β-estradiol was subcutaneously administered at the nape of the neck. 1 week after the administration, the amount of glucose (2-deoxy-D-glucose) intake of muscles (soleus muscle) (amount of glucose transport to muscles), and intramuscular expression / production levels of actin, P-IRβ, AKT, P-AKT and Glut4 (as a relative ratio with respect to the expression level of actin) were measured for Tg mice in Tx+E group (Tg: +) and in Ct group (Tg: -), and for WT mice in Tx+E group (WT: +) and in Ct group (WT: -) . The above-described P-IRβ, AKT, P-AKT, and Glut4 are molecules that are involved in glucose intake. The results are shown in Fig. 3. Results in Fig. 3B show relative ratios (folds) of an amount of glucose intake in presence of insulin with respect to an amount of glucose intake in absence of insulin, for WT mice in Ct group (represented as "WT: -" in the figure) and in Tx+E group (WT: +), and for Tg mice in Ct group (Tg: -) and in Tx+E group (Tg: +).

Fig. 3C is a western blotting image for measuring intramuscular expression levels of actin, P-IRβ, ANT, P-AKT, and Glut4 (represented as relative ratios with respect to an expression level of actin) for WT mice in Ct group (WT: -) and in Tx+E group (WT: +), and for Tg mice in Ct group (Tg: -) and in Tx+E group (Tg: +). Fig. 3D, F, G, and E are results comparing expression intensities of each of the molecules (P-IRβ, AKT, P-AKT, and Glut4) among each of the mice groups. Fig. 3H shows the results of immunostaining the expression of Glut4 in muscular tissues using the anti-Glut4 antibody for WT mice in Ct group (WT), and for Tg mice in Ct group (Tg) and in Tx+E group (Tx+E). Shown at the bottom of Fig. 3H are the results of semiquantifying the expression intensity of Glut4 in muscular tissues using LSM Zeiss software. The expression intensity was a result of a measurement in random fields (n = 30 / section) of muscular tissues, and was represented as intensity per pixel.

Fig. 3I is a schematic diagram showing signal transduction involved in glucose intake. As shown here, glucose intake is enhanced when expressions of AKT and Glut4, which are molecules involved in glucose intake, increase, and AKT and Glut4 become active.

From the above-described results, it was confirmed that, with diabetes model mice (Tg mice), the amount of glucose intake of muscles in Tx-E group administered with 17β-estradiol (E) significantly increases 20-40 weeks after the administration, when compared to the amount of glucose intake of muscles in Ct group that have not been administered with 17β-estradiol (E). In addition, glucose transporter (Glut4) increased in muscle cells of Tg mice in Tx-E group, indicating fine intracellular signaling. Thus, it was confirmed that administration of estradiol upregulates the expression of intramuscular AKT and Glut4, and consequently enhances the intake of sugar by muscles.

Fig. 3A shows the results obtained by: incubating, for 10 minutes at 30°C in KRB buffer containing 1 mM 2-deoxy-D-[$^3$H]glucose (1.5 μCi/ml) and 7 mM D-[$^{14}$C]mannitol (0.3 μCi/ml) (PerkinElmer Life Sciences), muscles (soleus muscles) of each mice (WT and Tg mice) in the estradiol administration group (represented as "Tx-E: +" in Fig. 3A) and in the control group (represented as "Tx-E: -" in Fig. 3A), in presence of insulin (represented as "Ins: +" in Fig. 3A) and in absence of insulin (represented as "Ins: -" in Fig. 3A); and measuring the amount (μmol/g/h) of glucose intake of muscles. It was indicated here that the experiment was a success, since the amount of glucose intake in presence (+) of insulin was increased in all of the mice.

Experimental Example 4: Effect of estradiol administration (II)

Tg23 mice (11 weeks of age) were divided into four groups (each group: n = 4 to 10): an estradiol administration group (E only group), a testectomy group (Tx only group), a testectomy-and-estradiol administration group (Tx+E group), and a control group (Tg group). For E only group, sham surgery of testectomy (Tx) was performed, and a single pellet of 17β-estradiol (E) was subcutaneously administered at the nape of the neck. For Tx only group, testectomy was performed, and a single pellet that consists of a vehicle and does not include 17β-estradiol was subcutaneously administered at the nape of the neck. For Tx+E group, testectomy was performed, and a single pellet of 17β-estradiol was subcutaneously administered at the nape of the neck. For Tg group, sham surgery of testectomy was performed, and a single pellet that consists of a vehicle and does not include 17β-estradiol was subcutaneously administered at the nape of the neck.

Then, blood glucose levels (mg/dl) were measured every week for E only group, Tx only group, Tx+E group, and Tg group, and wild-type mice (WT). In addition, measurements were performed with Tx+E group, Tg group, and wild-type mice (WT), for serum insulin concentrations (pg/mL), serum glucagon concentrations (pg/mL), blood urea nitrogen (BUN) concentrations (mg/dL), total serum cholesterol levels (g/dL), aspartate aminotransferase (AST) / alanine aminotransferase (ALT), and food consumption per day (g/day). Furthermore, oral glucose tolerance test was performed, and changes in blood glucose level and serum insulin concentration over time were measured.

The results are shown in Fig. 4A to I. The results show that the administration was effective for reducing blood glucose level in not only Tx+E, but also E only. In Tx+E mice, serum insulin level was also largely increased, and all of glucagon, BUN, total cholesterol, AST/ ALT, and food consumption returned to normal. Even with the oral glucose tolerance test, the blood glucose level returned to basal level after a time point of 180 minutes, and insulin secretion was also increased. These results indicate that blood glucose level and blood parameters were normalized.

Experimental Example 5: Effect of estradiol administration (III)

Tg23 mice (23-24 weeks of age) were divided into two groups (each group: n = 4 to 10): an estradiol administration group and a control group. For the estradiol administration group (Tx+E group), testectomy (Tx) was performed, and a single pellet of 17β-estradiol (E) was subcutaneously administered at the nape of the neck. For the control group (Tg group), sham surgery of testectomy was performed, and a single pellet (Innovative Research of America) that consists of a vehicle and does not include 17β-estradiol was subcutaneously administered. Then, blood glucose levels of these mice were monitored every week. The result is shown in Fig. 5A.

Shown in Fig. 5A are: change (indicated by a solid line in the figure) in blood glucose level of Tx+E group; change (indicated as a dotted line in the figure) in blood glucose level of Tg group; and blood glucose level (indicated as D in the figure) of wild-type mice (WT group). In addition, Fig. 5B and C respectively show area ($\mu m^2$) of β-cells on a single section of pancreatic tissues, and the number of pancreatic islets whose area of β-cells is 5001 $\mu m^2$ or larger, for each of the mice at the time of blood glucose level measurement (A: Tg group (55 weeks of age), B: Tx+E group at 1 week after estradiol administration, C: Tx+E group (55 weeks of age) at 42 weeks after estradiol administration, and D: WT group (55 weeks of age)).

All of the images in Fig. 5D show the results of immunostaining pancreases at a time point of B (1 week after estradiol administration) for Tx+E group using the anti-insulin antibody; and cells observed in the pancreatic duct epithelial cells and stained in brown are insulin-producing cells.

Fig. 5E shows the results of comparing the number of insulin-producing cells that were newly generated in the pancreatic duct epithelial cells of Tx+E group and Tg group (both 23-24 weeks of age).

Furthermore, Fig. 5F shows the results of observing the production of Pdx-1 (Pancreas duodenum homeobox 1) (indicated by white arrows in the figure) by immunostaining the pancreases of Tx+E group and Tg group (both 23-24 weeks old) using the anti-Pdx-1 antibody. In the figure, "d" represents a pancreatic duct epithelial cell (duct). Furthermore, Fig. 5G shows the results of comparing the numbers of Pdx-1 in the pancreases of Tx+E group and Tg group (both 23-24 weeks of age).

These results, in particular, the results from Fig. 5E and F, show that neogenesis of β-cells were enhanced in the group administered with estradiol (Tx+E group).

Sections of pancreatic tissues of mice in Tx+E group (Treatment) and in Tg group, and wild-type mice (WT) (all 48 weeks of age) were prepared and observed under an electron microscope. The results are shown in Fig. 6. As is obvious from these results, insulin secretory granules were observed in Tx+E group (Treatment). Thus, it can be understood that mature β-cells were formed in Treatment group, since insulin granules were generated in a manner similar to the wild-type mice (WT).

In addition, glomeruli and renal tubules were PAS-stained for mice in Tx+E group (Treatment) and in Tg group, and wild-type mice (WT) (all 48 weeks of age). Fig. 7A shows the results of comparing PAS-positive areas (pixels) in glomeruli, and Fig. 7B shows images of PAS-stained cells / tissues of renal tubules.

**Claims**

1. An insulin-producing cell inducer comprising an estrogenic compound as an active ingredient thereof.

2. The insulin-producing cell inducer according to claim 1, wherein the estrogenic compound is at least one member selected from the group consisting of estradiol or esters thereof, estrone, estriol or esters thereof, equilin, ethinylestradiol, conjugated estrogens, stilbestrol, and hexestrol.

3. A glucose intake enhancer that enhances glucose intake of muscles, the enhancer comprising an estrogenic compound as an active ingredient thereof.

4. The glucose intake enhancer according to claim 3, wherein the estrogenic compound is at least one member selected from the group consisting of estradiol or esters thereof, estrone, estriol or esters thereof, equilin, ethinylestradiol, conjugated estrogens, stilbestrol, and hexestrol.

5. A therapeutic agent for diabetes or a diabetes complication, the therapeutic agent comprising: the insulin-producing cell inducer according to claim 1 or 2 in an effective dose for inducing insulin-producing cells; or the glucose intake enhancer according to claim 3 or 4 in an effective dose for enhancing glucose intake of muscles.

6. The therapeutic agent according to claim 5, wherein the diabetes complication is diabetic nephropathy.

7. A method for inducing insulin-producing cells in a patient with diabetes or a diabetes complication, the method comprising a step of administering, to the patient with diabetes or the diabetes complication, the insulin-producing cell inducer according to claim 1 or 2 in an effective dose to induce insulin-producing cells.

8. A method for enhancing glucose intake of muscles in a patient with diabetes or a diabetes complication, the method comprising a step of administering, to the patient with diabetes or the diabetes complication, the glucose intake enhancer according to claim 3 or 4 in an effective dose to enhance glucose intake of muscles.

9. A therapeutic method for diabetes or a diabetes complication, the method comprising a step of administering, to a patient with diabetes or the diabetes complication, the insulin-producing cell inducer according to claim 1 or 2 in an effective dose to induce insulin-producing cells, or administering the glucose intake enhancer according to claim 3 or 4 in an effective dose to enhance glucose intake of muscles.

10. An estrogenic compound for use in a method for preventing or treating diabetes or a diabetes complication.

11. The estrogenic compound according to claim 10, wherein the estrogenic compound is at least one member selected from the group consisting of estradiol or esters thereof, estrone, estriol or esters thereof, equilin, ethinylestradiol, conjugated estrogens, stilbestrol, and hexestrol.

12. The estrogenic compound according to claim 10, wherein the diabetes complication is diabetic nephropathy.

Fig. 1

(2)

(1)

(3)

(4)

Fig. 2

Fig. 3

Fig. 4

Fig. 5

Fig. 6

Fig. 7

## INTERNATIONAL SEARCH REPORT

| | International application No. |
|---|---|
| | PCT/JP2010/062121 |

A. CLASSIFICATION OF SUBJECT MATTER
*A61K31/565*(2006.01)i, *A61P3/10*(2006.01)i, *A61P13/12*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K31/565, A61P3/10, A61P13/12, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | The Japanese Journal of Veterinary Science, 1989, Vol.51, No.4, pages 823-826 | 1-6,10-12 |
| X | EP 635264 A2 (Eli Lilly and Co.), 25 January 1995 (25.01.1995), | 1,3,5,6,10, 12 |
| A | entire text | 2,4,11 |
| | & JP 7-48252 A    & US 5567713 A | |
| X | WO 00/41701 A1 (Novo Nordisk A/S), 20 July 2000 (20.07.2000), entire text | 1-6,10-12 |
| | & JP 2002-534470 A    & US 6555530 B1 | |
| | & EP 1143979 A | |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 August, 2010 (02.08.10) | 17 August, 2010 (17.08.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2010/062121 |

C (Continuation).   DOCUMENTS CONSIDERED TO BE RELEVANT

| Category* | Citation of document, with indication, where appropriate, of the relevant passages | Relevant to claim No. |
|---|---|---|
| X | WO 01/001969 A1 (Endorecherche, Inc.), 11 January 2001 (11.01.2001), entire text & JP 2003-503446 A     & US 6710059 B1 & EP 1196163 A | 1-6,10-12 |
| X | US 4507289 A (Progenics, Inc.), 26 March 1985 (26.03.1985), entire text & EP 148436 A1 | 1-6,10-12 |
| X | WO 99/48502 A1 (Universidad Miguel Hernandez), 30 September 1999 (30.09.1999), entire text & ES 2135353 A | 1-6,10-12 |
| A | WO 2006/032026 A2 (PR Pharmaceuticals, Inc.), 23 March 2006 (23.03.2006), entire text & JP 2008-513375 A     & US 2008/0220069 A1 & EP 1791546 A | 1-6,10-12 |
| A | WO 2003/015704 A2 (University of Pittsburgh), 27 February 2003 (27.02.2003), entire text & JP 2005-501096 A     & US 2003/0050294 A1 & EP 1418876 A | 1-6,10-12 |

Form PCT/ISA/210 (continuation of second sheet) (July 2009)

| INTERNATIONAL SEARCH REPORT | International application No. |
|---|---|
| | PCT/JP2010/062121 |

**Box No. II**  **Observations where certain claims were found unsearchable (Continuation of item 2 of first sheet)**

This international search report has not been established in respect of certain claims under Article 17(2)(a) for the following reasons:

1. ☒ Claims Nos.: 7-9
because they relate to subject matter not required to be searched by this Authority, namely:
Claims 7 to 9 involve "methods for treatment of the human body or animal body by surgery or therapy".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III**  **Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004072282 A

**Non-patent literature cited in the description**

- *The Journal of Biological Chemistry,* 1999, vol. 274 (30), 21095-21103
- *Molecular and Cellular Biology,* 2004, vol. 24 (7), 2831-2841
- *Biochemical and Biophysical Research Communication,* 1998, vol. 253 (3), 712-718